(19) Europäisches Patentamt
European Patent Office
Office européen des brevets

(11) Veröffentlichungsnummer: **0 203 608**
**B2**

(12) # NEUE EUROPÄISCHE PATENTSCHRIFT

(45) Veröffentlichungstag der neuen Patentschrift: **06.09.89**

(51) Int. Cl.⁴: **C 07 C 69/734, A 01 N 37/38**

(21) Anmeldenummer: **86107332.8**

(22) Anmeldetag: **30.05.86**

(54) **Acrylsäureester und Fungizide, die diese Verbindungen enthalten.**

(30) Priorität: **30.05.85 DE 3519282**

(43) Veröffentlichungstag der Anmeldung:
**03.12.86 Patentblatt 86/49**

(45) Bekanntmachung des Hinweises auf die Patenterteilung:
**07.01.88 Patentblatt 88/1**

(45) Bekanntmachung des Hinweises auf die Entscheidung über den Einspruch:
**06.09.89 Patentblatt 89/36**

(84) Benannte Vertragsstaaten:
**AT BE CH DE FR GB IT LI NL SE**

(56) Entgegenhaltungen:
**EP-A- 0 001 740**
**EP-A- 0 117 412**
**EP-A- 0 178 826**

(73) Patentinhaber: **BASF Aktiengesellschaft, Carl-Bosch-Strasse 38, D-6700 Ludwigshafen (DE)**

(72) Erfinder: **Schirmer, Ulrich, Dr., Berghalde 79, D-6900 Heidelberg (DE)**
Erfinder: **Karbach, Stefan, Dr., Sperlinggasse 3, D-6700 Ludwigshafen (DE)**
Erfinder: **Pommer, Ernst-Heinrich, Dr., Berliner Platz 7, D-6703 Limburgerhof (DE)**
Erfinder: **Ammermann, Eberhard, Dr., Sachsenstrasse 3, D-6700 Ludwigshafen (DE)**
Erfinder: **Steglich, Wolfgang, Prof. Dr., Hobsweg 77, D-5300 Bonn-Roettgen (DE)**
Erfinder: **Schwalge, Barbara Agnes Maria, Tannenweg 9, D-5204 Lohmar 1 (DE)**
Erfinder: **Anke, Timm, Prof. Dr., Theodor-Heuss-Strasse 17, D-6750 Kaiserslautern (DE)**

EP 0 203 608 B2

## Beschreibung

Die vorliegende Erfindung betrifft neue Acrylsäureester und Fungizide, welche diese Verbindungen enthalten.

Es ist bekannt, N-Tridecyl-2,6-dimethylmorpholin oder seine Salze, z.B. das Acetat, als Fungizide zu verwenden (DE 1 164 152, 1 173 722). Ihre fungizide Wirkung ist jedoch in manchen Fällen ungenügend.

Es wurde nun gefunden, daß neue Acrylsäureester der Formel

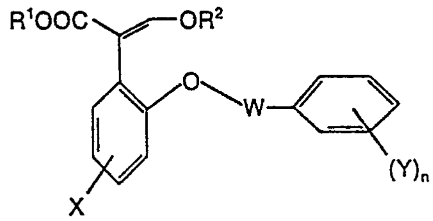

in der $R^1$ und $R^2$ unabhängig voneinander $C_1$–$C_8$-Alkyl bedeuten,

X Wasserstoff, $C_1$–$C_4$-Alkyl, Halogen, $C_1$–$C_4$-Alkoxy, Halogenalkyl, Cyano oder Nitro bedeutet,

W einen gegebenenfalls durch Alkyl substituierten, gegebenenfalls ungesättigten $C_1$–$C_{10}$-Alkylenrest bedeutet,

Y Wasserstoff, Alkyl, Halogenalkyl, Alkoxyalkyl, Cycloalkyl, Aralkyl, Aryl, Aryloxy, Halogen, eine gegebenenfalls substituierte an den Benzolrest annellierte $C_4H_4$-Kette, Alkoxy, Halogenalkoxy, Alkylthio, Thiocyanato, Cyano, $NO_2$, Aralkyloxy,

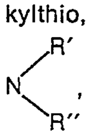 , NHCOR', 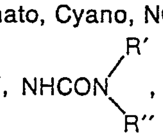 , COOR', 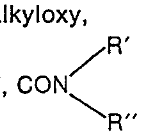

OSO$_2$R', SO$_2$R', COR', 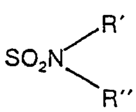

bedeutet, wobei R' und R'' jeweils unabhängig voneinander Wasserstoff, Alkyl, Alkoxy, Alkylthio, Cycloalkyl oder gegebenenfalls durch Alkyl, Halogen oder Alkoxy substituiertes Phenyl bedeuten und n die Zahlen 1 bis 4 bedeutet, außer den Verbindungen

α-(2-Benzyloxyphenyl)-β-methoxyacrylsäuremethylester,

α-[2-(2-Phenyl-prop-2-yloxy)-phenyl]-β-methoxyacrylsäuremethylester,

α-[2-(2-p-Chlorphenyl-prop-2-yloxy)-phenyl]-β-methoxyacrylsäuremethylester,

eine ausgezeichnete fungizide Wirkung haben.

Die in der allgemeinen Formel angeführten Reste können beispielsweise folgende Bedeutung haben:

$R^1$ bzw. $R^2$ gegebenenfalls verzweigtes $C_1$–$C_8$–Alkyl (z.B. Methyl, Ethyl, Isopropyl, n-Propyl, n-Butyl, sec.-Butyl, tert.-Butyl, iso-Butyl, sec.-Pentyl, n-Hexyl, α-Ethyl-n-hexyl, n-Octyl),

X Wasserstoff, $C_1$–$C_4$-Alkyl (z.B. Methyl, t-Butyl), Halogen, (z.B. Fluor, Chlor, Brom), $C_1$–$C_4$-Alkoxy (z.B. Methoxy, n-Butoxy) oder Halogenalkyl (z.B. Trifluormethyl), Cyan, Nitro,

W einen gegebenenfalls durch $C_1$–$C_4$-Alkyl substituierten, gegebenenfalls ungesättigten $C_1$–$C_{10}$-Alkylenrest (beispielsweise Methylen, Methylmethylen, Dimethylmethylen), Propylen, Allylen, Hexylen, Ethylen, Methylethylen, Methylpropylen, Ethylpropylen, Butylen, Pentylen, Methylpentylen, Dimethylpropylen, Heptylen, Ethylbutylen, Trimethylpentylen),

Y Wasserstoff, $C_1$–$C_{12}$-Alkyl (z.B. Methyl, Ethyl, t-Butyl, Dodecyl), Halogenalkyl (z.B. Trifluormethyl), $C_1$–$C_4$-Alkoxy-$C_1$–$C_4$-alkyl (z.B. Methoxymethyl), $C_5$–$C_8$-Cycloalkyl (z.B. Cyclohexyl), Aralkyl (z.B. Benzyl, Phenethyl), Aryl (z.B. Phenyl), Aryloxy (z.B. Phenoxy), Halogen (z.B. Fluor, Chlor, Brom oder Jod), eine gegebenenfalls substituierte $C_4$–$H_4$-Kette, die mit dem Benzolring zu einem gegebenenfalls substituierten Naphthylring kondensiert ist, $C_1$–$C_8$-Alkoxy (z.B. Isopropoxy, Hexyloxy), Halogen-$C_1$–$C_4$-alkoxy (z.B. 1,1,2,2-Tetrafluorethoxy), $C_1$–$C_4$-Alkylthio (z.B. Methylthio), Thiocyanato, Cyano, $NO_2$, Aralkyloxy (Benzyloxy, Phenethyloxy),

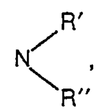 , 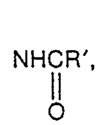 , 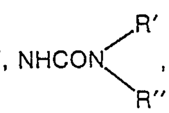 , COOR', 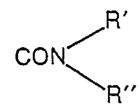

SO$_2$R', COR', OSO$_2$R', 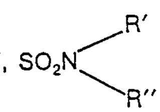

wobei R' und R'' jeweils unabhängig voneinander Wasserstoff, $C_1$–$C_4$-Alkyl (z.B. Methyl, Ethyl), $C_1$–$C_4$-Alkoxy (z.B. Methoxy, tert.-Butoxy), $C_1$–$C_4$-Alkylthio (z.B. Methylthio), $C_5$–$C_8$-Cycloalkyl (z.B. Cyclohexyl) oder gegebenenfalls durch $C_1$–$C_4$-Alkyl, Halogen oder $C_1$–$C_4$-Alkoxy substituiertes Phenyl (z.B. Phenyl, 3-Chlorphenyl, 4-Methylphenyl, 3-Methoxyphenyl) bedeuten.

Die neuen fungiziden Acrylsäureester können als E- oder Z-Isomere vorliegen. Die Stereoisomeren lassen sich beispielsweise durch Säulenchromatographie trennen oder aufgrund von Löslichkeitsunterschieden in reiner Form isolieren. Die isomerenreinen Verbindungen lassen sich durch bekannte Methoden in die jeweils anderen Isomere überführen. Die isomerenreinen Verbindungen werden wie ihre Mischungen von der vorliegenden Erfindung umfaßt. Für die Anwendung der neuen Verbindungen als Fungizide sind sowohl die Isomerengemische als auch die einheitlichen isomeren Verbindungen geeignet.

Die neuen Verbindungen können beispielsweise nach folgendem Verfahren hergestellt werden:

Ein Phenylessigsäureester der allgemeinen Formel

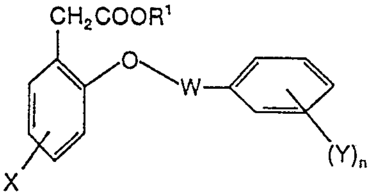

in der $R^1$, X, W und $(Y)_n$ die oben genannten Bedeutungen haben, wird mit Ameisensäuremethylester und Natriumhydrid in einem inerten Lösungsmittel

zur Reaktion gebracht. Die so erhaltenen Verbindungen der allgemeinen Formel

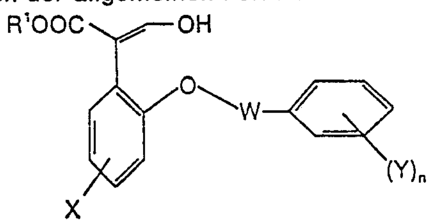

in der $R^1$, X, W und $(Y)_n$ die oben genannten Bedeutungen haben, werden mit einem Alkylierungsmittel in Gegenwart einer Base in einem inerten Lösungsmittel (z.B. Aceton) zu den neuen Verbindungen der allgemeinen Formel umgesetzt,

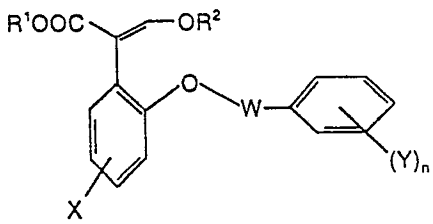

in der $R^1$, $R^2$, X, W und $(Y)_n$ die oben genannten Bedeutungen haben [Wislicenus, Liebigs Annalen 413, 206 (1917) und 424, 215 (1921)].

Die Phenylessigester der allgemeinen Formel

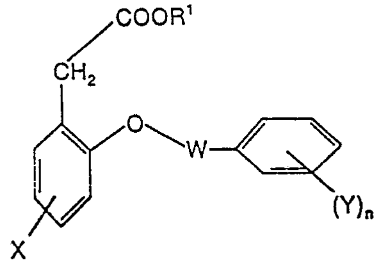

erhält man z.B. durch Reaktion von gegebenenfalls substituierten 2-Hydroxyphenylessigsäureestern mit Verbindungen des Typs

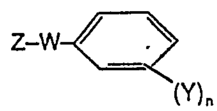

wobei $R^1$, X, W und $(Y)_n$ die oben genannten Bedeutungen haben und Z für eine Austrittsgruppe steht, z.B. Chlor, Brom, Jod, Mesylat, Tosylat [vgl. Houben-Weyl, Methoden der organischen Chemie VI/3, 54ff (1965)].

In entsprechender Weise lassen sich folgende Verbindungen herstellen:

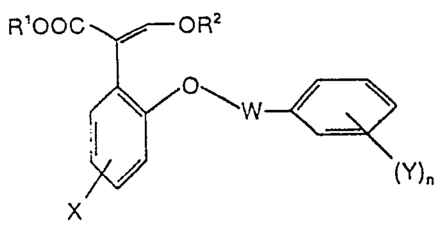

| Nr. | $R^1$ | $R^2$ | X | W | $(Y)_n$ | Fp°C/NMR/Isomeres |
|---|---|---|---|---|---|---|
| 2 | $CH_3$ | $CH_3$ | H | $-CH_2-CH_2-$ | H | |
| 3 | $CH_3$ | $CH_3$ | H | $-CH_2-CH=CH-$ | H | Harz |
| 4 | $CH_3$ | $CH_3$ | H | $-CH_2-CH_2-CH_2-$ | H | Harz |
| 5 | $CH_3$ | $CH_3$ | H | $-(CH_2)_5-$ | H | |
| 6 | $CH_3$ | $CH_3$ | H | $-(CH_2)_7-$ | H | |
| 7 | $CH_3$ | $CH_3$ | H | $-(CH_2)_8-$ | H | |
| 8 | $CH_3$ | $CH_3$ | H | $-(CH_2)_{10}-$ | H | |
| 9 | $CH_3$ | $CH_3$ | H | $-CH(CH_3)-$ | H | |
| 10 | $CH_3$ | $CH_3$ | H | $-CH(CH_3)-$ | 4-Cl | |
| 11 | $CH_3$ | $CH_3$ | H | $-CH(CH_3)-$ | $4-OCH_3$ | |
| 13 | $CH_3$ | $CH_3$ | H | $-C(CH_3)_2-$ | $3-CF_3$ | |
| 14 | $CH_3$ | $CH_3$ | H | $-CH_2CH(CH_3)CH_2-$ | H | |
| 15 | $CH_3$ | $CH_3$ | H | $-CH_2CH(CH_3)CH_2-$ | $4-t-C_4H_9$ | |
| 16 | $CH_3$ | $CH_3$ | H | $-CH_2CH(CH_3)CH_2-$ | $4-CH_3$ | |
| 17 | $CH_3$ | $CH_3$ | H | $-CH_2CH(CH_3)CH_2-$ | 4-Cl | |
| 18 | $CH_3$ | $CH_3$ | H | $-CH_2CH(CH_3)CH_2-$ | 4-F | |
| 19 | $CH_3$ | $CH_3$ | H | $-CH_2CH(CH_3)CH_2-$ | $3,4-(CH_3)_2$ | |
| 20 | $CH_3$ | $CH_3$ | H | $-CH(CH_3)CH_2-$ | H | |
| 21 | $CH_3$ | $CH_3$ | H | $-CH_2-CH(CH_3)-$ | H | |
| 22 | $CH_3$ | $CH_3$ | H | $-CH_2-CH_2-$ | $4-CH_3$ | |
| 23 | $CH_3$ | $CH_3$ | H | $-CH_2-CH_2-$ | 4-Cl | |
| 24 | $CH_3$ | $CH_3$ | H | $-CH_2-(CH=CH)_2-$ | H | |
| 25 | $CH_3$ | $CH_3$ | H | $-CH_2-C\equiv C-$ | H | |
| 26 | $CH_3$ | $CH_3$ | H | $-CH_2-$ | 2-Cl | |
| 27 | $CH_3$ | $CH_3$ | H | $-CH_2-$ | $2,4-Cl_2$ | 100-102 (Z), 116 (E) |
| 28 | $CH_3$ | $CH_3$ | H | $-CH_2-$ | 3-Cl | 57 (E) |
| 29 | $CH_3$ | $CH_3$ | H | $-CH_2-$ | $3,4-Cl_2$ | 107 (Z), 131 (E) |

| Nr. | $R^1$ | $R^2$ | X | W | $(Y)_n$ | Fp°C/NMR/Isomeres |
|---|---|---|---|---|---|---|
| 30 | $CH_3$ | $CH_3$ | H | $-CH_2-$ | 4–Cl | |
| 31 | $CH_3$ | $CH_3$ | H | $-CH_2-$ | 3,5-$Cl_2$ | |
| 32 | $CH_3$ | $CH_3$ | H | $-CH_2-$ | 2,4,5–$Cl_3$ | |
| 33 | $CH_3$ | $CH_3$ | H | $-CH_2-$ | 4–Br | 118 (E) |
| 34 | $CH_3$ | $CH_3$ | H | $-CH_2-$ | 4–F | 100 (E) |
| 35 | $CH_3$ | $CH_3$ | H | $-CH_2-$ | 4–$OCH_3$ | |
| 36 | $CH_3$ | $CH_3$ | H | $-CH_2-$ | 4–O–n $(C_4H_9)$ | |
| 37 | $CH_3$ | $CH_3$ | H | $-CH_2-$ | 4–O–t $(C_4H_9)$ | |
| 38 | $CH_3$ | $CH_3$ | H | $-CH_2-$ | 4–$CH_3$ | 71–74 (Z) 97–98 (E) |
| 39 | $CH_3$ | $CH_3$ | H | $-CH_2-$ | 2–$CH_3$ | |
| 40 | $CH_3$ | $CH_3$ | H | $-CH_2-$ | 3–$CH_3$ | |
| 41 | $CH_3$ | $CH_3$ | H | $-CH_2-$ | 4–t–$C_4H_9$ | |
| 42 | $CH_3$ | $CH_3$ | H | $-CH_2-$ | 4–cyclo–$C_6H_{11}$ | |
| 43 | $CH_3$ | $CH_3$ | H | $-CH_2-$ | 3–Benzyl | |
| 44 | $CH_3$ | $CH_3$ | H | $-CH_2-$ | 3–Phenoxy | |
| 45 | $CH_3$ | $CH_3$ | H | $-CH_2-$ | 4–Phenoxy | |
| 46 | $CH_3$ | $CH_3$ | H | $-CH_2-$ | 3–$CH_2OCH_3$ | |
| 47 | $CH_3$ | $CH_3$ | H | $-CH_2-$ | 2–$CH_2OCH_3$ | |
| 48 | $CH_3$ | $CH_3$ | H | $-CH_2-$ | 3–$CF_3$ | |
| 49 | $CH_3$ | $CH_3$ | H | $-CH_2-$ | 4–$CF_3$ | |
| 50 | $CH_3$ | $CH_3$ | H | $-CH_2-$ | 3–$C_2H_5$ | |
| 51 | $CH_3$ | $CH_3$ | H | $-CH_2-$ | 2–$OCH_3$ | |
| 52 | $CH_3$ | $CH_3$ | H | $-CH_2-$ | 3–$OCH_3$ | Harz (Z) |
| 53 | $CH_3$ | $CH_3$ | H | $-CH_2-$ | 4–J | |
| 54 | $CH_3$ | $CH_3$ | H | $-CH_2-$ | 3–F | |
| 55 | $CH_3$ | $CH_3$ | H | $-CH_2-$ | 3,4 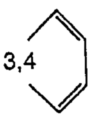 | |
| 56 | $CH_3$ | $CH_3$ | H | $-CH_2-$ | 2,3 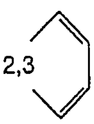 | |
| 57 | $CH_3$ | $CH_3$ | H | $-CH_2-$ | 4–$OCHF_2$ | |
| 58 | $CH_3$ | $CH_3$ | H | $-CH_2-$ | 3–$OCF_2CHF_2$ | |
| 59 | $CH_3$ | $CH_3$ | H | $-CH_2-$ | 4–$SCH_3$ | |
| 60 | $CH_3$ | $CH_3$ | H | $-CH_2-$ | 4–CN | Harz (E) |
| 61 | $CH_3$ | $CH_3$ | H | $-CH_2-$ | 3–CN | |
| 62 | $CH_3$ | $CH_3$ | H | $-CH_2-$ | 4–SCN | |
| 63 | $CH_3$ | $CH_3$ | H | $-CH_2-$ | 4–$N(CH_3)_2$ | |
| 64 | $CH_3$ | $CH_3$ | H | $-CH_2-$ | 3–$NHCOCH_3$ | |
| 65 | $CH_3$ | $CH_3$ | H | $-CH_2-$ | 3–$NHCOOCH_3$ | |
| 66 | $CH_3$ | $CH_3$ | H | $-CH_2-$ | 3–$NHCON(CH_3)_2$ | |
| 67 | $CH_3$ | $CH_3$ | H | $-CH_2-$ | 4–$COOCH_3$ | |
| 68 | $CH_3$ | $CH_3$ | H | $-CH_2-$ | 4–$CONHCH_3$ | |
| 69 | $CH_3$ | $CH_3$ | H | $-CH_2-$ | 4–$CON(CH_3)_2$ | |
| 70 | $CH_3$ | $CH_3$ | H | $-CH_2-$ | 4–$SO_2CH_3$ | |
| 71 | $CH_3$ | $CH_3$ | H | $-CH_2-$ | 4–Phenylsulfonyl | |
| 72 | $CH_3$ | $CH_3$ | H | $-CH_2-$ | 3–$COCH_3$ | |
| 73 | $CH_3$ | $CH_3$ | H | $-CH_2-$ | 4–$OSO_2CH_3$ | |
| 74 | $CH_3$ | $CH_3$ | H | $-CH_2-$ | 4–$SO_2N(CH_3)_2$ | |
| 75 | $CH_3$ | $CH_3$ | H | $-CH_2-$ | 4–CONH– 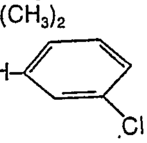 | |
| 76 | $CH_3$ | $CH_3$ | H | $-CH_2-$ | 4–Benzoyl | |
| 77 | $C_2H_5$ | $CH_3$ | H | $-CH_2-$ | H | |
| 78 | i–$C_3H_7$ | $CH_3$ | H | $-CH_2-$ | H | |
| 79 | n–$C_6H_{13}$ | $CH_3$ | H | $-CH_2-$ | H | |
| 80 | n–$C_4H_9$ | $CH_3$ | H | $-CH_2-$ | H | |
| 81 | n–$C_3H_7$ | $CH_3$ | H | $-CH_2-$ | H | |

| Nr. | $R^1$ | $R^2$ | X | W | $(Y)_n$ | Fp°C/NMR/Isomeres |
|---|---|---|---|---|---|---|
| 82 | $s-C_4H_9$ | $CH_3$ | H | $-CH_2-$ | H | |
| 83 | $CH_3$ | $C_2H_5$ | H | $-CH_2-$ | H | Harz (E) |
| 84 | $CH_3$ | $i-C_3H_7$ | H | $-CH_2-$ | H | 73-74 (E) |
| 85 | $CH_3$ | $n-C_6H_{13}$ | H | $-CH_2-$ | H | Harz (E) |
| 86 | $CH_3$ | $n-C_3H_7$ | H | $-CH_2-$ | H | |
| 87 | $CH_3$ | $s-C_4H_9$ | H | $-CH_2-$ | H | |
| 88 | $CH_3$ | $CH_3$ | $3-CH_3$ | $-CH_2-$ | H | |
| 89 | $CH_3$ | $CH_3$ | $4-CH_3$ | $-CH_2-$ | H | |
| 90 | $CH_3$ | $CH_3$ | 5-Cl | $-CH_2-$ | H | |
| 91 | $CH_3$ | $CH_3$ | 5-Br | $-CH_2-$ | H | |
| 92 | $CH_3$ | $CH_3$ | 6-Cl | $-CH_2-$ | H | |
| 93 | $CH_3$ | $CH_3$ | $6-CH_3$ | $-CH_2-$ | H | |
| 94 | $CH_3$ | $CH_3$ | 5-F | $-CH_2-$ | H | |
| 95 | $CH_3$ | $CH_3$ | $5-CF_3$ | $-CH_2-$ | H | |
| 96 | $CH_3$ | $CH_3$ | H | $-CH_2-$ | $3-NO_2$ | |
| 97 | $CH_3$ | $CH_3$ | H | $-CH_2-$ | $3-N(CH_3)_2$ | |
| 98 | $CH_3$ | $CH_3$ | H | $-CH_2CH(CH_3)CH_2$ | 4-Phenoxy | |
| 99 | $CH_3$ | $CH_3$ | H | $-CH_2CH(CH_3)CH_2$ | 2-F | |
| 101 | $CH_3$ | $CH_3$ | H | $CH_2C(CH_3)_2$ | H | |
| 102 | $CH_3$ | $CH_3$ | H | $CH(CH_3)_2$ | $2,5(CH_3)_2$ | |
| 103 | $CH_3$ | $CH_3$ | H | $-CH_2-$ | $4NO_2$ | |
| 104 | $CH_3$ | $CH_3$ | H | $-CH_2-$ | 2Cl, 6F | |
| 105 | $CH_3$ | $CH_3$ | H | $-CH_2-$ | $2,3,6\ (Cl)_3$ | 100 (Z), 90 (E) |
| 106 | $CH_3$ | $CH_3$ | H | $-CH_2-$ | $3,4(CH_3)_2$ | |
| 107 | $CH_3$ | $CH_3$ | H | $-CH_2-$ | $2-CH_3$, $4t-C_4H_9$ | |
| 108 | $CH_3$ | $CH_3$ | H | $-CH_2-$ | $2,4(CH_3)_2$ | 79 (E) |
| 109 | $CH_3$ | $CH_3$ | H | $-CH_2-$ | $2-NO_2$ | |
| 110 | $CH_3$ | $CH_3$ | H | $-CH_2-$ | $4-C_{12}H_{25}$ | |
| 111 | $CH_3$ | $CH_3$ | H | $-CH_2-$ | $4-C_2H_5$ | |
| 112 | $CH_3$ | $CH_3$ | H | $-CH_2-$ | $3,4,5\ (OCH_3)_3$ | |
| 113 | $CH_3$ | $CH_3$ | H | $-Ch_2-$ | $2,5(CH_3)_2$ | |
| 114 | $CH_3$ | $CH_3$ | H | $-CH_2-$ | 2-F | Harz (Z) |
| 115 | $CH_3$ | $CH_3$ | H | $-CH_2-$ | 2CN | |
| 116 | $CH_3$ | $CH_3$ | H | $-CH_2-$ | $2,6-Cl_2$ | 83-85 (Z) |
| 117 | $CH_3$ | $CH_3$ | H | $-CH_2-$ | 2-Cl, 6CN | |
| 118 | $CH_3$ | $CH_3$ | H | $-CH_2-$ | 2-Cl, $4NO_2$ | |
| 119 | $CH_3$ | $CH_3$ | H | $-CH_2-$ | $2,4(NO_2)_2$ | |
| 120 | $CH_3$ | $CH_3$ | H | $-(CH_2)_5-$ | 4-Cl | |
| 121 | $CH_3$ | $n-C_4H_9$ | H | $-CH_2$ | H | 73-74 (E) |
| 122 | $CH_3$ | $CH_3$ | H | $-CH_2-$ | 3-Benzyloxy | |
| 123 | $CH_3$ | $CH_3$ | H | $-CH_2-$ | 3-Phenethyl | |
| 124 | $CH_3$ | $CH_3$ | H | $-CH_2-$ | 4-Benzyloxy | |
| 125 | $CH_3$ | $CH_3$ | H | $-CH_2-$ | 4-Phenethyl | |
| 126 | $CH_3$ | $CH_3$ | H | $-CH_2-$ | 3-Phenethyloxy | |
| 127 | $CH_3$ | $CH_3$ | H | $-CH_2-$ | 4-Phenethyloxy | |

Die neuen Verbindungen zeichnen sich, allgemein ausgedrückt, durch eine hervorragende Wirksamkeit gegen ein breites Spektrum von pflanzenpathogenen Pilzen, insbesondere aus der Klasse der Ascomyceten, Phycomyceten und Basidiomyceten, aus. Sie sind zum Teil systemisch wirksam und können als Blatt- und Bodenfungizide eingesetzt werden.

Besonders interessant sind die fungizide Verbindungen für die Bekämpfung einer Vielzahl von verschiedenen Kulturpflanzen oder ihren Samen, insbesondere Weizen, Roggen, Gerste, Hafer, Reis, Mais, Baumwolle, Soja, Kaffee, Zuckerrohr, Obst und Zierpflanzen im Gartenbau, Weinbau sowie Gemüse – wie Gurken, Bohnen und Kürbisgewächse.

Die neuen Verbindungen sind insbesondere geeignet zur Bekämpfung folgender Pflanzenkrankheiten:

Erysiphe graminis (echter Mehltau) in Getreide,
Erysiphe cichoracearum und Sphaerotheca fuliginea an Kürbisgewächsen,
Podosphaera leucotricha an Äpfeln,
Uncinulca necator an Reben,
Puccinia-Arten an Getreide,
Rhizoctonia solani an Baumwolle,
Ustilago-Arten an Getreide und Zuckerrohr,
Venturia inaequalis (Schorf) an Äpfeln,
Septoria nodorum an Weizen,
Pyrenophora teres an Gerste,
Botrytis cinerea (Grauschimmel) an Erdbeeren, Reben,

Cercospora arachidicola an Erdnüssen,
Pseudocercosporella herpotrichoides an Weizen, Gerste,
Pyricularia oryzae an Reis,
Phytophthora infestans an Kartoffeln und Tomaten,
Alternaria solani an Kartoffeln, Tomaten,
Plasmopara viticola an Reben sowie Fusarium- oder Verticillium-Arten an verschiedenen Pflanzen.

Die Verbindungen werden angewendet, indem man die Pflanzen mit den Wirkstoffen besprüht oder bestäubt oder die Samen der Pflanzen mit den Wirkstoffen behandelt. Die Anwendung erfolgt vor oder nach der Infektion der Pflanzen oder Samen durch Pilze.

Die neuen Substanzen können in die üblichen Formulierungen übergeführt werden, wie Lösungen, Emulsionen, Suspensionen, Stäube, Pulver, Pasten und Granulate. Die Anwendungsformen richten sich ganz nach den Verwendungszwekken; sie sollen in jedem Fall eine feine und gleichmässige Verteilung der wirksamen Substanz gewährleisten. Die Formulierungen werden in bekannter Weise hergestellt, z.B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel als Hilfslösungsmittel verwendet werden können. Als Hilfsstoffe kommen dafür im wesentlichen in Frage: Lösungsmittel wie Aromaten (z.B. Xylol, Benzol), chlorierte Aromaten (z.B. Chlorbenzole), Paraffine (z.B. Erdölfraktionen), Alkohole (z.B. Methanol, Butanol), Amine (z.B. Ethanolamin, Dimethylformamid) und Wasser; Trägerstoffe wie natürliche Gesteinsmehle, z.B. Kaoline, Tonerden, Talkum, Kreide und synthetische Gesteinsmehle (z.B. hochdisperse Kieselsäure, Silikate); Emulgiermittel, wie nichtionogene und anionische Emulgatoren (z.B. Polyoxyethylen-Fettalkohol-Ether, Alkylsulfonate und Arylsulfonate) und Dispergiermittel, wie Lignin, Sulfitablaugen und Methylcellulose.

Die fungiziden Mittel enthalten im allgemeinen zwischen 0,1 und 95, vorzugsweise zwischen 0,5 und 90 Gew.% Wirkstoff.

Die Aufwandmengen liegen je nach Art des gewünschten Effektes zwischen 0,05 und 3 kg Wirkstoff oder mehr je ha. Die neuen Verbindungen können auch im Materialschutz u.a. zur Bekämpfung holzzerstörender Pilze wie Coniophora puteana und Polystictus versicolor eingesetzt werden. Die neuen Wirkstoffe können auch als fungizid wirksame Bestandteile öliger Holzschutzmittel zum Schutz von Holz gegen holzverfärbende Pilze eingesetzt werden. Die Anwendung erfolgt in der Weise, daß man das Holz mit diesen Mitteln behandelt, beispielsweise tränkt oder anstreicht.

Die Mittel bzw. die daraus hergestellten gebrauchsfertigen Zubereitungen, wie Lösungen, Emulsionen, Suspensionen, Pulver, Stäube, Pasten oder Granulate werden in bekannter Weise angewendet, beispielsweise durch Versprühen, Vernebeln, Verstäuben, Verstreuen, Beizen oder Gießen.

Die erfindungsgemäßen Mittel können in diesen Anwendungsformen auch zusammen mit anderen Wirkstoffen vorliegen, wie z.B. Herbiziden, Insektiziden, Wachstumsregulatoren und Fungiziden, oder auch mit Düngemitteln vermischt und ausgebracht werden. Beim Vermischen mit Fungiziden erhält man dabei in vielen Fällen eine Vergrößerung des fungiziden Wirkungsspektrums.

Die folgende Liste von Fungiziden, mit denen die erfindungsgemäßen Verbindungen kombiniert werden können, soll die Kombinationsmöglichkeiten erläutern, nicht aber einschränken.

Fungizide, die mit den erfindungsgemäßen Verbindungen kombiniert werden können, sind beispielsweise:

Schwefel,
Dithiocarbamate und deren Derivate, wie
Ferridimethyldithiocarbamat,
Zinkdimethyldithiocarbamat,
Zinkethylenbisdithiocarbamat,
Manganethylenbisdithiocarbamat,
Mangan-Zink-ethylendiamin-bis-dithiocarbamat,
Tetramethylthiuramdisulfide,
Ammoniak-Komplex von Zink-(N,N-ethylen-bis-dithiocarbamat),
Ammoniak-Komplex von Zink-(N,N-propylen-bis-dithiocarbamat),
Zink-(N,N'-propylen-bis-dithiocarbamat),
N,N'-Propylen-bis-(thiocarbamoyl)-disulfid;
Nitroderivate, wie
Dinitro-(1-methylheptyl)-phenylcrotonat,
2-sec-Butyl-4,6-dinitrophenyl-3,3-dimethylacrylat,
2-sec-Butyl-4,6-dinitrophenyl-isopropylcarbonat,
5-Nitro-isophthalsäure-di-isopropylester,
heterocyclische Strukturen, wie
2-Heptadecyl-2-imidazolin-acetat,
2,4-Dichlor-6-(o-chloranilino)-s-triazin,
O,O-Diethyl-phthalimidophosphonothioat,
5-Amino-1-(bis-(dimethylamino)-phosphinyl)-3-phenyl-1,2,4-triazol,
2,3-Dicyano-1,4-dithioanthrachinon,
2-Thio-1,3-dithio-(4,5-b)-chinoxalin,
1-(Butylcarbamoyl)-2-benzimidazol-carbaminsäuremethylester,
2-Methoxycarboxylamino-benzimidazol,
2-(Furyl)-(2)-benzimidazol,
2-(Thiazolyl)-(4)-benzimidazol,
N-(1,1,2,2-Tetrachlorethylthio)-tetrahydrophthalimid,
N-Trichlormethylthio-tetrahydrophthalimid,
N-Trichlormethylthio-phthalimid,
N-Dichlorfluormethylthio-N',N'-Dimethyl-N-phenyl-schwefelsäureamid,
5-Ethoxy-3-trichlormethyl-1,2,3-thiadiazol,
2-Rhodanmethylthiobenzthiazol,
1,4-Dichlor-2,5-dimethoxybenzol,
4-(2-Chlorphenylhydrazono)-3-methyl-5-isoxazolon,
Pyridin-2-thio-oxid,
8-Hydroxychinolin bzw. dessen Kupfersalz,
2,3-Dihydro-5-carboxanilido-6-methyl-1,4-oxathiin-4,4-dioxid,

2,3-Dihydro-5-carboxanilido-6-methyl-1,4-oxathiin,
2-Methyl-5,6-dihydro-4H-pyran-3-carbonsäure-anilid,
2-Methyl-furan-3-carbonsäureanilid,
2,5-Dimethyl-furan-3-carbonsäureanilid,
2,4,5-Trimethyl-furan-3-carbonsäureanilid,
2,5-Dimethyl-furan-3-carbonsäurecyclohexyl-amid,
N-Cyclohexyl-N-methoxy-2,5-dimethyl-furan-3-carbonäureamid,
2-Methyl-benzoesäure-anilid,
2-Jod-benzoesäure-anilid,
N-Formyl-N-morpholin-2,2,2-trichlorethyl-acetal,
Piperazin-1,4-diylbis-1-(2,2,2-trichlor-ethyl)-formamid,
1-(3,4-Dichloranilino)-1-formylamino-2,2,2-trichlorethan,
2,6-Dimethyl-N-tridecyl-morpholin bzw. dessen Salze,
2,6-Dimethyl-N-cyclododecyl-morpholin bzw. dessen Salze N-[3-(p-tert.-Butylphenyl)-2-methylpropyl]-cis-2,6-dimethylmorpholin,
N-[3-(p-tert.-Butylphenyl)-2-methylpropyl]-piperidin,
1-[2-(2,4-Dichlophenyl)-4-ethyl-1,3-dioxolan-2-yl-ethyl]-1H-1,2,4-triazol,
1-[2-(2,4-Dichlorphenyl)-4-n-propyl-1,3-dioxolan-2-yl-ethyl]-1H-1,2,4-triazol,
N-(n-Propyl)-N-(2,4,6-trichlorphenoxyethyl)-N'-imidazol-yl-harnstoff,
1,(4-Chlorphenoxy)-3,3-dimethyl-1-(1H-1,2,4-triazol-1-yl)-2-butanon,
1-(4-Chlorphenoxy)-3,3-dimethyl-1-(1H-1,2,4-triazol-1-yl)-2-butanol,
α-(2-Chlorphenyl)-α(4-chlorphenyl)-5-pyrimidin-methanol,
5-Butyl-2-dimethylamiono-4-hydroxy-6-methyl-pyrimidin,
Bis-(p-Chlorphenyl)-pyridinmethanol,
1,2-Bis-(3-ethoxycarbonyl-2-thioureido)-benzol,
1,2-Bis-(3-methoxycarbonyl-2-thioureido)-benzol,
sowie verschiedene Fungizide, wie
Dodecylguanidinacetat,
3-(3-(3,5-Dimethyl-2-oxycyclohexyl)-2-hydroxyethyl)-glutarimid,
Hexachlorbenzol,
DL-Methyl-N-(2,6-dimethyl-phenyl)-N-furoyl(2)-alaninat,
DL-N-(2,6-Dimethyl-phenyl)-N-(2'-methoxyacetyl)-alanin-methylester,
N-(2,6-Dimethylphenyl)-N-chloracetyl-D,L-2-aminobutyrolacton,
DL-N-(2,6-Dimethylphenyl)-N-(phenylacetyl)-alaninmethylester,
5-Methyl-5-vinyl-3-(3,5-dichlorphenyl)-2,4-dioxo-1,3-oxazolidin,
3-(3,5-Dichlorphenyl)5-methyl-5-methoxymethyl-2,3-oxazolidin-2,4-dion,
3-(3,5-Dichlorphenyl)-1-isopropylcarbamoyl-hydantoin,
N-(3,5-Dichlorphenyl)-1,2-dimethylcyclopropan-1,2-dicarbonsäureamid,
2-Cyano-N-(ethylaminocarbonyl)-2-methoximino-acetamid,
1-(2-(2,4-Dichlorphenyl)-pentyl)-1H-1,2,4-triazol,
2,4'-Difluor-α-(1H-1,2,4-triazolyl-1-methyl)-benz-hydrylalkohol,
N-(3-Chlorphenyl)-5-trifluormeth-pyridin,
N-(2,6-Dinitrophenyl)-3-chlor-pyridin,
N-(4-Trifluormethyl-phenyl)-2-amino-pyridin,
1-(bis(4-Fluorphenyl)methylsilyl)-methyl-1H-1,2,4-triazol.

Für die folgenden Versuche wurde als Vergleich die bekannten Wirkstoffe N-Tridecyl-2,6-dimethyl-morpholin (A) und sein Acetat (B) verwendet.

Anwendungsbeispiel 1

Wirksamkeit gegen Weizenmehltau

Blätter von in Töpfen gewachsenen Weizenkeimlingen der Sorte «Frühgold» werden mit wässeriger Spritzbrühe, die 80% Wirkstoff und 20% Emulgiermittel in der Trockensubstanz enthält, besprüht und 24 Stunden nach dem Antrocknen des Spritzbelages mit Oidien (Sporen) des Weizenmehltaus (Erysiphe graminis var. tritici) bestäubt. Die Versuchspflanzen werden anschließend im Gewächshaus bei Temperaturen zwischen 20 und 22 °C und 75 bis 80% relativer Luftfeuchtigkeit aufgestellt. Nach 7 Tagen wird das Ausmaß der Mehltauentwicklung ermittelt.

Das Ergebnis des Versuches zeigt, daß die Wirkstoffe Nr. 28 und 114 bei der Anwendung als 0,025; 0,006 und 0,0015%ige (Gew.%) Spritzbrühe eine bessere fungizide Wirkung (90%) zeigen als die bekannten Wirkstoffe A und B (50%).

Anwendungsbeispiel 3

Wirksamkeit gegen Pyrenophora teres

Blätter von in Töpfen gewachsenen Gerstenkeimlingen der Sorte «Asse» werden im Zweiblattstadium mit wässeriger Spritzbrühe, die 80% Wirkstoff und 20% Emulgiermittel in der Trockensustanz enthält, bis zur Tropfnässe besprüht. Am folgenden Tage werden die angetrockneten Pflanzen mit einer wässerigen Sporensuspension von Pyrenophora teres inoculiert und für 7 Tage bei 17 bis 19 °C und 95% relativer Luftfeuchtigkeit weiter kultiviert. Dann wird das Ausmaß des Pilzbefalls ermittelt.

Das Ergebnis des Versuches zeigt, daß die Wirkstoffe Nr. 4, 28, 52 und 114 bei der Anwendung als 0,05%ige Spritzbrühe eine bessere fungizide Wirkung zeigen (90%) als der bekannte Wirkstoff A (60%).

Anwendungsbeispiel 5

Wirksamkeit gegen Plasmopara viticola

Blätter von Topfreben der Sorte «Müller Thurgau» werden mit wässeriger Spritzbrühe, die 80% Wirkstoff und 20% Emulgiermittel in der Trockensubstanz enthält, besprüht. Um die Wirkungsdauer der Wirkstoffe beurteilen zu können, werden die Pflanzen nach dem Antrocknen des Spritzbelages 8 Tage im Gewächshaus aufgestellt. Erst dann werden die Blätter mit einer Zoosporenaufschwemmung von Plasmopara viticola (Rebenperonospora) infiziert. Danach werden die Reben zunächst für 48 Stunden in einer wasserdampfgesättigten Kammer bei 24 °C und anschließend für 5 Tage in einem Gewächshaus mit Temperaturen zwischen 20 und 30 °C aufgestellt. Nach dieser Zeit werden die Pflanzen zur Beschleunigung des Sporangienträgerausbruches abermals für 16 Stunden in der feuchten Kammer aufgestellt. Dann erfolgt die Beurteilung des Ausmasses des Plizausbruches auf den Blattunterseiten.

Das Ergebnis des Versuches zeigt, daß die Wirkstoffe Nr. 3, 4, 28, 33, 52, 60, 114 und 116 bei der Anwendung als 0,05%ige Spritzbrühe eine bessere fungizide Wirkung zeigen (97%) als der bekannte Wirkstoff A (50%).

## Patentansprüche

1. Acrylsäureester der allgemeinen Formel

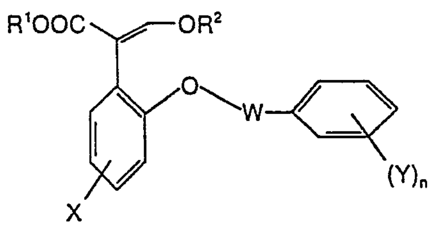

in der $R^1$ und $R^2$ unabhängig voneinander $C_1$–$C_8$-Alkyl bedeuten,

X Wasserstoff, $C_1$–$C_4$-Alkyl, Halogen, $C_1$–$C_4$-Alkoxy, Halogenalkyl, Cyano oder Nitro bedeutet,

W einen gegebenenfalls durch Alkyl substituierten, gegebenenfalls ungesättigten $C_1$–$C_{10}$-Alkylenrest bedeutet,

Y Wasserstoff, Alkyl, Halogenalkyl, Alkoxyalkyl, Cycloalkyl, Aralkyl, Aryl, Aryloxy, Halogen, eine gegebenenfalls substituierte an den Benzolrest annellierte $C_4H_4$-Kette, Alkoxy, Halogenalkoxy, Alkylthio, Thiocyanato, Cyano, $NO_2$, Aralkyloxy,

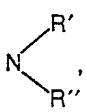 , NHCOR', 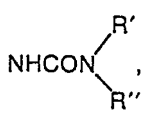 , COOR', 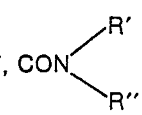

OSO$_2$R', SO$_2$R', COR', 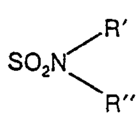

bedeutet, wobei R' und R'' jeweils unabhängig voneinander Wasserstoff, Alkyl, Alkoxy, Alkylthio, Cycloalkyl oder gegebenenfalls durch Alkyl, Halogen oder Alkoxy substituiertes Phenyl bedeuten und n die Zahlen 1 bis 4 bedeutet, außer den Verbindungen

α-(2-Benzyloxyphenyl)-β-methoxyacrylsäuremethylester,

α-[2-(2-Phenyl-prop-2-yloxy)-phenyl]-β-methoxyacrylsäuremethylester,

α-[2-(2-p-Chlorphenyl-prop-2-yloxy)-phenyl]-β-methoxyacrylsäuremethylester,

2. Fungizid, enthaltend eine Verbindung gemäß Anspruch 1-

3. Verfahren zur Bekämpfung von Pilzen, dadurch gekennzeichnet, daß man die Pilze oder die von Pilzbefall bedrohten Materialien, Pflanzen, Saatgüter oder den Erdboden mit einer fungizid wirksamen Mengen einer Verbindung gemäß Anspruch 1 behandelt.

4. Fungizid, enthaltend einen festen oder flüssigen Trägerstoff und einen Acrylsäureester der allgemeinen Formel

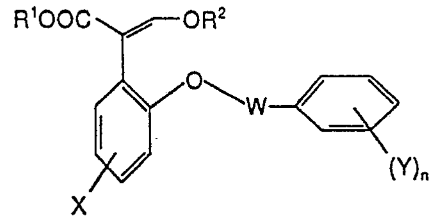

in der $R^1$ und $R^2$ unabhängig voneinander $C_1$–$C_8$-Alkyl bedeuten,

X Wasserstoff, $C_1$–$C_4$-Alkyl, Halogen, $C_1$–$C_4$-Alkoxy, Halogenalkyl, Cyano oder Nitro bedeutet,

W einen gegebenenfalls durch Alkyl substituierten, gegebenenfalls ungesättigte $C_1$–$C_{10}$-Alkylenrest bedeutet,

Y Wasserstoff, Alkyl, Halogenalkyl, Alkoxyalkyl, Cycloalkyl, Aralkyl, Aryl, Aryloxy, Halogen, eine gegebenenfalls substituierte an den Benzolrest annellierte $C_4H_4$-Kette, Alkoxy, Halogenalkoxy, Alkylthio, Thiocyanato, Cyano, $NO_2$, Aralkyloxy,

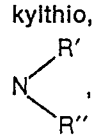 , 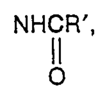 , 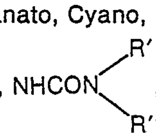 , COOR', 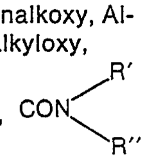

SO$_2$R', COR', OSO$_2$R', 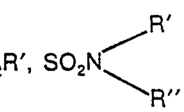

bedeutet, wobei R' und R'' jeweils unabhängig voneinander Wasserstoff, Alkyl, Alkoxy, Alkylthio, Cycloalkyl oder gegebenenfalls durch Alkyl, Halogen oder Alkoxy substituiertes Phenyl bedeuten und n die Zahlen 1 bis 4 bedeutet, außer den Verbindungen

α-(2-Benzyloxyphenyl)-β-methoxyacrylsäuremethylester,

α-[2-(2-Phenyl-prop-2-yloxy)-phenyl]-β-methoxyacrylsäuremethylester,

α-[2-(2-p-Chlorphenyl-prop-2-yloxy)-phenyl]-β-methoxyacrylsäuremethylester,

## Claims

1. An acrylate of the formula

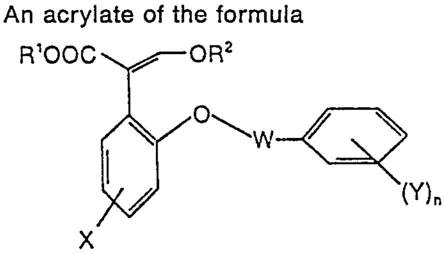

where $R^1$ and $R^2$ independently of one another are each $C_1$–$C_8$-alkyl, X is hydrogen, $C_1$–$C_4$-alkyl, halogen, $C_1$–$C_4$-alkoxy, haloalkyl, cyano or nitro, W is unsubstituted or alkyl-substituted, saturated or unsaturated $C_1$–$C_{10}$-alkylene, Y is hydrogen, alkyl, haloalkyl, alkoxyalkyl, cycloalkyl, aralkyl, aryl, aryloxy, halogen, an unsubstituted or substituted

$C_4H_4$ chain fused to the benzene radical, alkoxy, haloalkoxy, alkylthio, thiocyanato, cyano, $NO_2$, aralkoxy,

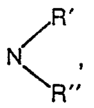, NHCOR', 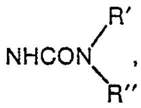, COOR', 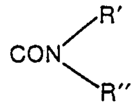

OSO$_2$R', SO$_2$R', COR', 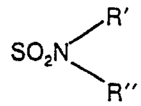

and R' and R'' independently of one another are each hydrogen, alkyl, alkoxy, alkylthio or cycloalkyl or are each phenyl which is unsubstituted or substituted by alkyl, halogen or alkoxy, and n is from 1 to 4, excluding the compounds
methyl α-(benzyloxyphenyl-β-methoxyacrylate,
methyl α-[2-(2-phenylprop-2-yloxy)-phenyl]-β-methoxyacrylate,
methyl α-[2-(2-p-chlorophenylprop-2-yloxy)-phenyl]-β-methoxyacrylate.

2. A fungicide containing a compound as claimed in claim 1.

3. A process for combating fungi, wherein the fungi or the materials, plants, seed or the soil threatened by fungal attack are treated with a fungicidally, effective amount of a compound as claimed in claim 1.

4. A fungicide containing a solid or liquid carrier and an acrylate of the formula

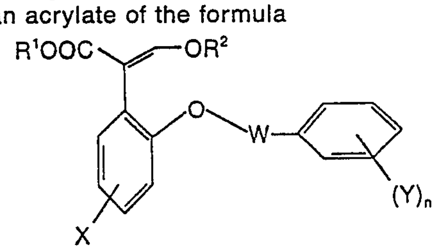

where $R^1$ and $R^2$ independently of one another are each $C_1$–$C_8$-alkyl, X is hydrogen, $C_1$–$C_4$-alkyl, halogen, $C_1$–$C_4$-alkoxy, haloalkyl, cyano or nitro, W is unsubstituted or alkyl-substituted, saturated or unsaturated $C_1$–$C_{10}$-alkylene, Y is hydrogen, alkyl, haloalkyl, alkoxyalkyl, cycloalkyl, aralkyl, aryl, aryloxy, halogen, an unsubstituted or substituted $C_4H_4$ chain fused to the benzene radical, alkoxy, haloalkoxy, alkylthio, thiocyanato, cyano, $NO_2$, aralkyloxy,

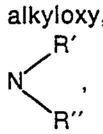, 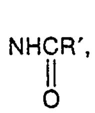, 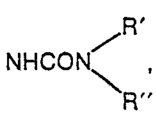, COOR', 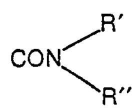

SO$_2$R', COR', OSO$_2$R', 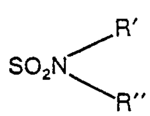

and R' and R'' independently of one another are each hydrogen, alkyl, alkoxy, alkylthio or cycloalkyl or are each phenyl which is unsubstituted or substituted by alkyl, halogen or alkoxy, and n is from 1 to 4, excluding the compounds
methyl α-(benzyloxyphenyl)-β-methoxcyacrylate,
methyl α-[2-(2-phenylprop-2-yloxy)-phenyl]-b-methoxyacrylate,
methyl α-[2-(2-p-chlorophenylprop-2-yloxy)-phenyl]-β-methoxyacrylate.

## Revendications

1. Ester acrylique de formule générale

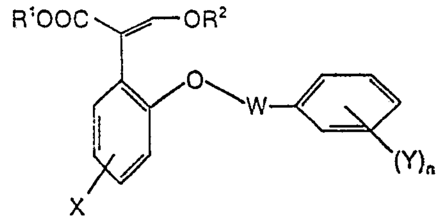

dans laquelle $R^1$ et $R^2$ représentent, indépendamment l'un de l'autre, alkyle en $C_1$–$C_8$, X représente hydrogène, alkyle en $C_1$–$C_4$, halogène, alcoxy en $C_1$–$C_4$, halogénoalkyle, cyano ou nitro,
W représente un reste alkylène en $C_1$–$C_{10}$ éventuellement insaturé, éventuellement substitué par alkyle,
Y représente hydrogène, alkyle, halogénoalkyle, alkoxyalkyle, cycloalkyle, aralkyle, aryle, aryloxy, halogène, une chaîne en $C_4H_4$, condensée, éventuellement substituée sur le reste benzène, alcoxy, halogénoalcoxy, alkylthio, thiocyanato, cyano, $NO_2$, aralkyloxy,

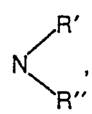, NHCOR', 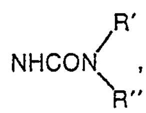, COOR', 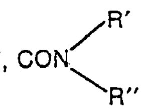

OSO$_2$R', SO$_2$R', COR', 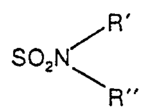

R' et R'', représentent chacun, indépendamment l'un de l'autre, hydrogène, alkyle, alcoxy, alkylthio, cycloalkyle ou phényle, éventuellement substitué par alkyle, halogène ou alcoxy et n représente les nombre 1 à 4, à l'exception des composés suivants:

ester méthylique de l'acide α-(2-benzyloxyphényle)-β-méthoxyacrylique
ester méthylique de l'acide α-[2-(2-phényle-prop-2-yloxy)-phényl]-β-méthoxyacrylique
ester méthylique de l'acide α-[2-(2-p-chlorophényle-prop-2-yloxy)-phényl]-β-méthoxyacrylique.

2. Fongicide contenant un composé selon la revendication 1.

3. Procédé de lutte contre les champignons, caractérisé par le fait que l'on traite les champignons, ou les matériaux, plantes, semences ou le sol, menacés par l'attaque par champignons, avec une quantité efficace, du point de vue fongicide, d'un composé selon la revendication 1.

4. Fongicide contenant un véhicule solide ou liquide et un ester acrylique de formule générale

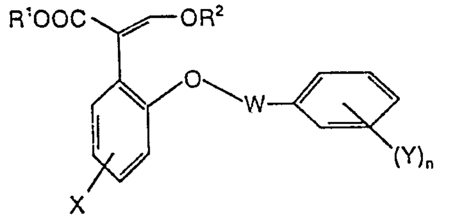

dans laquelle $R^1$ et $R^2$ représentent, indépendamment l'un de l'autre, alkyle en $C_1$–$C_8$, X représente hydrogène, alkyle en $C_1$–$C_4$, halogène, alcoxy en $C_1$–$C_4$, halogénoalkyle, cyano ou nitro,

W représente un reste alkylène en $C_1$–$C_{10}$ éventuellement insaturé, éventuellement substitué par alkyle,

Y représente hydrogène, alkyle, halogénoalkyle, alcoxyalkyle, cycloalkyle, aralkyle, aryle, aryloxy, halogène, une chaîne en $C_4H_4$, condensée, éventuellement substituée sur le reste benzène, alcoxy, halogénoalcoxy, alkylthio, thiocyanato, cyano, $NO_2$, aralkyloxy

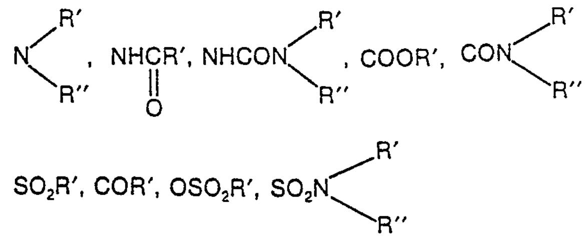

, COOR', ,

$SO_2R'$, COR', $OSO_2R'$,

R' et R'' représentent chacun, indépendamment l'un de l'autre, hydrogène, alkyle, alcoxy, alkylthio, cycloalkyle ou phényle, éventuellement substitué par alkyle, halogène ou alcoxy et n représente les nombres 1 à 4, à l'exception des composés suivants:

ester méthylique de l'acide α-(2-benzyloxyphényle)-β-méthoxyacrylique

ester méthylique de l'acide α-[2-(2-phényle-prop-2-yloxy)-phényl]-β-méthoxyacrylique

ester méthylique de l'acide α-[2-(2-p-chlorophényle-prop-2-yloxy)-phényl]-β-méthoxyacrylique.